Europäisches Patentamt

European Patent Office

Office européen des brevets

(11) Publication number: **0 225 709 B1**

# EUROPEAN PATENT SPECIFICATION

(45) Date of publication of patent specification: **27.05.92**

(51) Int. Cl.⁵: **G01N 33/577**, G01N 33/574, G01N 33/68, //G01N33/543

(21) Application number: **86308212.9**

(22) Date of filing: **22.10.86**

(54) Immunometric assay for high molecular weight carcinoembryonic antigen.

(30) Priority: **22.10.85 US 790261**

(43) Date of publication of application:
**16.06.87 Bulletin 87/25**

(45) Publication of the grant of the patent:
**27.05.92 Bulletin 92/22**

(84) Designated Contracting States:
**AT BE CH DE FR GB IT LI LU NL SE**

(56) References cited:
**EP-A- 0 062 892**
**EP-A- 0 098 162**
**EP-A- 0 105 714**
**EP-A- 0 188 093**
**US-A- 4 098 876**

**HYBRIDOMA, vol. 2, no. 3, 1983, pages 329-339, US; M. HERLYN et al.: "Detection of carcinoembryonic antigen and related antigens in sera of patients with gastrointestinal tumors using monoclonal antibodies in double-determinant radioimmunoassays"**

(73) Proprietor: **CENTOCOR, INC.**
**244 Great Valley Parkway**
**Malvern, PA 19355(US)**

(72) Inventor: **Schoemaker, Hubert J.P.**
**495 School House Lane**
**Devon, PA 19333(US)**
Inventor: **Brennan, Suzanne E.**
**326 Apple Drive**
**Exton, PA 19341(US)**
Inventor: **Schlom, Jeffrey**
**10525 Tyler Terrace**
**Potomac, MD 20854(US)**
Inventor: **Brock, Paul**
**1098 Birnam Place**
**West Chester, PA 19380(US)**

(74) Representative: **Holdcroft, James Gerald, Dr. et al**
**Graham Watt & Co., Riverhead**
**Sevenoaks, Kent TN13 2BN(GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

CLINICAL CHEMISTRY, vol. 31, no. 2, February 1985, pages 191-195, Winston-Salem, North-Carolina, US; Y.-S.V. LIU et al.: "A more specific, simpler radioimmunoassay for carcinoembryonic antigen with use of monoclonal antibodies"

CANCER RESEARCH, vol. 44, January 1984, pages 245-253, US; M. BLASZCZYK et al.: "Characterization of gastrointestinal tumor-associated carcinoembryonic antigen-related antigens defined by monoclonal antibodies"

## Description

Field of the Invention

This invention is in the field of immunochemistry.

Background

Carcinoembryonic antigen (CEA) is a tumor-associated antigen usually present in patients with tumors of the colon. Gold and Freeman first described CEA as a component of colon cancer. See Gold P., et al., J. Exp. Med., 121, 439-462. CEA has since been investigated in a variety of other types of cancer. See Goldenberg D.M. et al., Ann. Intern. Med. 94, 407-409 (1981); Go, V.L.W. and Zamcheck N., Cancer 50, 2618-2623 (1982).

CEA assays have been produced using polyclonal antibodies as well as monoclonal antibodies against low molecular weight CEA components. These assays have several drawbacks:

(1) the sensitivity is insufficient to detect reliably subclinical cancer;

(2) the assays often fail to distinguish between malignant and non-malignant diseases; and

(3) assays based upon polyclonal antibodies typically show crossreactivity against a range of CEA of different molecular weights. (Problems associated with this phenomenon are crossreactivity against normal cross reactive antigens (NCA) and other low molecular weight substances. See Herlyn M., et al., Hybridoma 23, 329-339 (1983); Shively J.E. et al., (Stewart Sell, ed.) Humana Press, Clifton, N.J., (1980)-).

Because of these drawbacks, CEA assays cannot be used to diagnose cancer in "healthy" (i.e. not having colon cancer) appearing individuals. Presently, available CEA assays are used primarily to predict recurrence in colorectal cancer patients. More sensitive and specific CEA assays are needed to improve current clinical utility and to allow CEA assays to be used for diagnoses of cancer in "healthy" appearing individuals.

Disclosure of the Invention

This invention pertains to improved solid phase immunometric assays for quantitative determination of high molecular weight (180kD) carcinoembryonic antigen and to diagnostic test kits containing the reagents for performance of the the assays. The assays are of the sandwich or "double antibody" type which employ a unique combination of two monoclonal antibodies against high molecular weight CEA, the 1116NS-3d (3d6 antibody) and the COL-1 antibody. One of the antibodies, 3d6, is employed to form a solid phase adsorbent; the other, COL-1, is employed as a labeled antibody.

Sandwich immunometric assays utilizing the 3d6 and COL-1 can be of the forward, reverse, or simultaneous type. In the forward type, the sample to be tested is reacted with a solid phase adsorbent containing the 3d6 antibody. During incubation, CEA in the sample is bound to the solid phase. Thereafter, a labeled antibody, COL-1, is reacted with adsorbed CEA to form a ternary complex of labeled antibody-CEA-adsorbed antibody. The label associated with the solid phase is directly proportional to the amount of CEA in the sample.

In the reverse format, a labeled antibody is incubated with the sample to form soluble CEA-antibody complexes. In the second step, the solid phase is used to adsorb the labeled complex and the solid phase is analyzed for bound label.

In the simultaneous mode, the solid phase immunoadsorbent, the labeled antibody and the sample are incubated together. After the single incubation, the solid phase is analyzed for associated label.

Commercial kits containing reagents for performance of the assays can include the solid phase immunoadsorbent containing the 3d6 antibody, a solution of labeled COL-1 antibody and optionally, CEA standards.

Brief Description of the Drawings

Figure 1 shows flow cytometric analysis of anti-CEA monoclonal antibody binding to the surface of human polymorphonuclear leukocytes and (inset) reactivity of COL-1 antibody with colon carcinoma extract versus spleen extract

Figure 2 shows immunodetection of CEA by monoclonal antibodies COL-1 to -15 using Western blotting analysis.

3

Figure 3 shows immunodetection by Western blotting of CEA and CEA-related antigens in extract of human tissues.

Figure 4 is a comparison of standard curves for the 3d6/COL-1 immunoenzymetric assay and the Abbott CEA-EIA Monoclonal test.

Best Mode of Carrying Out the Invention

The assays for CEA employ a unique combination of two anti-CEA monoclonal antibodies. The first is the 3d6 (1116NS-3d) antibody described by Koprowski et al., U.S. Patent 4,349,528, the teachings of which are incorporated by reference herein. This antibody is specific for the 180,000 Dalton (180KD) molecular weight form of CEA. The second antibody is the COL-1 antibody was prepared as described in detail infra by Dr. Jeffrey Schlom of the National Cancer Institute/NIH, Bethesda, Maryland. COL-1 is a murine antibody of the G subclass 2a and is also specific for the 180 KD form of CEA. The COL-1 antibody and the COL-1 hybridoma have been deposited at the American Type Culture Collection, Rockville, Maryland and assigned identification number SD857. The term "CEA" as used herein means the 180KD form of the molecule.

The assays of this invention are sandwich or "double antibody" immunometric assays in which antigen (CEA) is measured directly by reacting it with an excess of labeled antibody. In such assays, CEA is insolubilized on a solid phase immunoadsorbent which specifically binds to CEA. Depending on the assay format, CEA is insolubilized either before or after the reaction with labeled anti-CEA antibody. The immunoadsorbent is formed by affixing the 3d6 monoclonal antibody to an appropriate solid phase support. The labeled antibody is the COL-1 antibody. The label associated with the solid phase is directly proportional to the amount of CEA in the sample.

The sandwich immunometric assays can be performed in forward, reverse or simultaneous mode. In the forward mode, the 3d6 and COL-1 monoclonal antibodies provide the basis for an extremely specific and sensitive sandwich immunometric assay for 180KD CEA. The 3d6 antibody is used to form the immunoadsorbent and the COL-1 antibody serves as the labeled antibody (tracer), and the assay is performed as outlined below. With these two antibodies, the assay is specific for high molecular weight CEA and is highly sensitive; levels of CEA in plasma or serum samples at limiting concentrations of 0.25 ng/ml can be detected. This represents at least a twofold increase in sensitivity over existing assays for CEA.

In a forward sandwich assay for CEA, the 3d6 antibody is affixed to a solid phase to form an immunoadsorbent specific for CEA. A liquid sample to be tested for CEA is incubated with the immunoadsorbent. Incubation is maintained for a sufficient period of time to allow the CEA in the liquid sample to bind to the solid phase immunoadsorbent. After incubation, the solid phase immunoadsorbent is separated from the incubation mixture. The immunoadsorbent may be washed to remove unbound CEA and interfering substances, such as non-specific binding proteins, which may also be present in the liquid sample. The immunoadsorbent containing CEA bound to immobilized antibody is subsequently incubated with labeled COL-1 antibody. Again, the incubation is carried out for a period of time and under conditions sufficient to ensure binding of the labeled antibody to CEA bound to the immunoadsorbent. After the second incubation, another wash may be performed to remove unbound labeled antibody from the solid phase immunoadsorbent. The labeled antibody bound to the solid phase immunoadsorbent is then measured, and the amount of labeled antibody detected serves as an indication of the level of CEA in the liquid sample.

In reverse mode, an incubation solution is formed of the liquid sample to be tested and the soluble, labeled COL-1 antibody. After appropriate incubation, the solution is contacted with the solid phase immunoadsorbent containing the 3d6 antibody. After another incubation, the immunoadsorbent is separated from the mixture (and generally washed) and the label bound to the immunoadsorbent is taken as an indication of the amount of CEA in the liquid sample.

In the simultaneous mode, an incubation mixture is formed of the liquid sample, the labeled anti-CEA antibody and the solid phase immunoadsorbent. When antibodies which react with different epitopes are employed, they do not compete for binding. After appropriate incubation, the solid phase immunoadsorbent is separated from the mixture and the label associated with the immunoadsorbent is measured to give an indication of the amount of CEA in the liquid sample.

For each incubation step in the various formats of the assays, the time and conditions of incubation are selected to ensure maximal binding of CEA to the immobilized antibody and to labeled antibody. In the forward version of the assay where two incubation steps are required, the solid phase immunoadsorbent containing immobilized CEA antibody is incubated with the liquid sample for about 1-4 hours at room temperature to 45°C in order to obtain maximal binding. The optimal duration of the subsequent incubation with labeled antibody is also about 1-4 hours at temperatures ranging from room temperature to 45°C. The entire assay may be run in about two (2) hours. The conditions and parameters which yield maximal binding of CEA may be established for the other formats of the assay by no more than routine experimentation.

In the various solid phase assays of this invention, the immunoadsorbent is separated from incubation mixtures containing the liquid sample, the labeled antibody or both. Separation can be accomplished by any conventional separation technique such as sedimentation or centrifugation. Preferably, though not necessarily, the immunoadsorbent is washed prior to contacting it, when required, with a second incubation medium and prior to measuring the amount of label associated with the immunoadsorbent. Generally, the washing is performed three times with water. The washing removes nonspecific interfering substances or excess labeled antibody which may affect the accuracy and sensitivity of the assay.

The immunoassays of this invention are used to detect and quantify CEA in a liquid sample. Liquid samples include essentially all biological fluids such as blood, or components of blood such as plasma or serum and urine, lymph, etc.

The solid phase immunoadsorbents employed in the assays of this invention are formed by coupling 3d6 anti-CEA antibodies to a solid phase support. Many types of solid-phases may be employed. Well-known solid phases include beads formed of glass, polystyrene, polypropylene, dextran, and other materials; tubes formed from or coated with such materials, etc. The antibody can be either covalently or noncovalently bound to the solid-phase by techniques such as covalent bonding via an amide or ester linkage or adsorption. Those skilled in the art will know many other suitable solid-phases and methods for immobilizing antibodies thereon, or will be able to ascertain such using no more than routine experimentation.

The COL-1 monoclonal antibody can be labeled in several ways for use in the assays. The antibodies can be labeled with a radioactive material, such as $I^{125}$; labeled with an optical label, such as a fluorescent material; labeled with an enzyme such as horse radish peroxidase (HRP), alkaline phosphatase, galactosidase, et.; or labeled by some other technique. The antibody can also be biotinylated and labeled avidin can be used to detect binding to the immunoadsorbent.

Methods of labeling the anti-CEA antibodies with $I^{125}$ include the Chloramine T technique and the Bolton-Hunter technique. Antibodies can be labeled with enzyme by direct or indirect conjugation of the enzyme to the antibody.

In an alternate format of the assays, the second, soluble antibody may be employed without a label. A labeled antibody against the second antibody can be used to detect bindings to the immunoadsorbents. For example, a labeled anti-(murine IgG) antibody can be used to assess COL-1 binding to the immunoadsorbent.

To determine the amount of CEA in the liquid sample, either the amount of label associated with the immunoadsorbent or the amount of unbound labeled antibody, that is, labeled antibody which remains in soluble form, is measured. Generally, it is preferable to measure the label bound to the immunoadsorbent because at very low concentrations of antigen, only small amounts of labeled antibody bind to the immunoadsorbent. Thus, for accuracy the label associated with the immunoadsorbent should be measured directly. The label may be detected by a gamma counter, for example, if the label is a radioactive gamma emitter, or by a fluorimeter, for example, if the label is a fluorescent material. In the case of an enzyme label, detection can be done by colorimetric methods employing a competing substrate for the enzyme.

The measured amount of label detected is then compared to a pre-established quantitative relationship between the amount of label and the amount of CEA. The quantitative relationship can be determined by performing the assay with standard liquid samples containing known amounts of CEA. For several samples containing different amounts of CEA, the assay is conducted and the amount of label either bound or unbound to the immunoadsorbent is determined; a curve is constructed defining the quantitative relationship between the amount of label and the amount of CEA. By reference to the curve, the amount of CEA in a liquid sample containing an unknown amount of CEA can be determined from the amount of label detected.

The 3d6 and COL-1 monoclonal antibody can be produced in large quantity for use in the assay by injecting the antibody-secreting hybridoma cells into the peritoneal cavity of mice (primed with mineral oil) and harvesting the ascites fluid into which antibody is secreted. The antibody can be purified from the ascites fluid by affinity chromatography. Purified antibody can be stored until use in a borate buffered saline solution containing a preservative such as 0.1% sodium azide.

The reagents for performing the assays of this invention may be assembled in assay kits. For instance, a kit might comprise a solid phase immunoadsorbent containing the 3d6 antibody, a solution of labeled COL-1 monoclonal antibody and, optionally, a CEA standard. The COL-1 and 3d6 monoclonal antibodies are suitable for kits because reagents formed with the antibodies are stable over a significant period of time when stored.

A particularly preferred form of the assay is a solid phase immunoenzymetric assay (IEMA) as detailed below. The 3d6 antibody is affixed to a polystyrene bead by, for example, physical adsorption (e.g. 6 mg

5

antibody per bead 1/4" in diameter). Beads coated with 3d6 can be stored for at least 6 months in dry form and provided for use in the assay. The COL-1 antibody is labeled with HRP by, for example, direct conjugation.

To perform the assay, the polystryrene bead is incubated with a sample of patient serum or plasma. The incubation is run for about one hour at 45°C. The bead is then separated from the sample and washed (e.g. 3 times with water). The bead is then incubated with a solution of the COL-1-HRP conjugate for about one hour at 45°C. Thereafter, the bead is removed from the solution, washed as above and the enzyme label associated with the bead is assessed. Measurement of HRP activity associated with the bead can be done by conventional colorimetric techniques. For example, the bead is incubated with the substrate O-Phenylenediamine-2HCl (OPD) for about 15-30 minutes at room temperature. The enzymatic reaction is halted by addition of 1N sulfuric acid and the amount of converted substrate is determined by measurement of optical density of the reaction solution at 492nm. The optical density is proportional to the concentration of CEA in the sample.

Kits for performance of the IEMA can include the following reagents:

a. container(s) of polystyrene beads coated with 3d6 antibody; and

b. container(s) of a solution of COL-1 antibody labeled with HRP.

Other components of the kit can be:

c. container(s) of CEA standards, e.g. human serum based solutions e.g. (20% serum) containing CEA ranging in concentration from 0-120 ng/ml;

d. a positive CEA control e.g. human serum containing CEA at 10 ng/ml; and

e. the HRP substrate.

The 3d6/COL-1 assay of this invention has several advantages. Most importantly, the assay shows a low false positive rate as indicated by low CEA values detected in normal individuals, in smokers and in persons with benign colon diseases. Based upon statistical distribution of CEA values in these patient groups, the assay has diagnostic value for colorectal cancer.

Additionally, the assay is very sensitive. Levels of CEA as low as 0.25 ng/ml can be reliably detected. Further, there is no need to extract plasma or serum samples because the 3d6 and COL-1 antibody are specific for the high molecular weight form of CEA.

The invention is illustrated further by the following exemplification.

Exemplification

Preparation of COL-1 Antibody

Cell Extract and Membrane Preparation

The neoplastic and normal human adult tissues were obtained from Departments of Surgery and Pathology, George Washington University, Washington, D.C. Cell extracts and partially purified membranes of tissues and cell lines were prepared as described by Colcher, D. et al. Cancer Res., 41:1451-1459 (1981). Protein concentrations were determined by the method of Lowry et al., J. Biol. Chem., 193: 265-275 (1951).

Immunizations

Four-wk-old BALB/c mice were immunized by i.p. injection of 100 $\mu$g of colon carcinoma cell extract or membrane-enriched fraction, emulsified in an equal volume of complete Freund's adjuvant. Seven days later mice were given an additional i.p. inoculation of 100 $\mu$g of immunogen emulsified in an equal volume of incomplete Freund's adjuvant. After an additional 7 days, mice were boosted i.v. with 10 $\mu$g of immunogen, and spleens were removed for fusing 3 days later.

The immunogen used for the generation of MAbs COl-9, -12, -13, and -15 was an extract of a human colon carcinoma metastasis to lymph node. The immunogen used for the generation of MAb COL-2 was an extract of a primary human colon carcinoma. The immunogen used for the generation of MAbs COL-3, -5, -6, -7, -8, -10, and -14 was partially purified membrane preparation of the primary colon carcinoma used in the above protocol. Extracts of two colon carcinoma metastases (one lymph node and one spleen) and an extract of a primary colon carcinoma were used as sequential immunogens for the generation of MAbs COL-1, -4, and -11. One hundred $\mu$g of the extract of the lymph node metastasis were used as the primary immunogen followed in sequence by immunizations with 100 $\mu$g of the spleen metastasis and primary carcinoma extracts. The final i.v. boost consisted of a mixture of 10 $\mu$g of each of both metastases and the primary carcinoma extracts.

## Hybridoma Methodology and Monoclonal Antibodies

Somatic cell hybrids were prepared using the method of Herzenberg et al., Cell hybrids of myelomas with antibody forming cells and T lymphocytes with T cells In: D. M. Weir (ed.), Handbook of Experimental Immunology, pp. 25.1-25.7. Oxford Backwell Scientific Publications, 1978, with some modifications. Colcher et al. supra. All hybridoma cell lines were cloned twice by limiting dilution. All fifteen characterized hybridoma cultures were shown to be negative for the presence of Mycoplasma. Immunoglobulin isotypes were determined as described. Colcher et al. supra. A single pool of hybridoma tissue culture supernatant from each MAb was used for all experiments described below. Thus MAbs titer differences can be ruled out in the comparison of one experiment to another. An anti-HCEA, and IgGl, was purchased from Hybritech, Inc., La Jolla, CA. MAb B1.1, an IgG2a, is reactive with CEA and CEA-related determinants and was described previously Colcher, D. et al. Monoclonal antibodies to human mammary carcinoma associated antigens and their potential uses for diagnosis, prognosis, and therapy. In: Immunoadiagnostics, pp. 215-258, 1983.

## Cells

The BALB/c nonsecreting myeloma cell line, P3-NS1-Ag4-1 (NS-1), was obtained from Dr. J. Kim, NIH, Bethesda, MD. The WiDr and LS-174T colon carcinoma cell lines were obtained from the American Type Tissue Culture Collection, Rockville, MD. The HT-29 colon carcinoma cell line and breast carcinoma cell lines MCF-7 and BT-20 were obtained from the Breast Cancer Task Force, National Cancer Institute, NIH, Bethesda, MD. The A204 rhabdomyosarcoma cell line and bladder carcinoma cell lines HA-698 and HA-1054 were obtained from Dr. S. Aaronson, National Cancer Institute, NIH, Bethesda, MD. Cell line WiDr-51 is a subset of cells from the WiDR colon carcinoma cell line which was derived by repeated fluorescence-activated cell sorting of WiDr with anti-CEA MAb Bl.1 All cell lines were maintained on the growth medium recommended by their respective sources. Purified CEA from five different colon cancer patients was provided by Dr. H. Hansen, Hoffman-La Roche, Nutley, NJ. One CEA preparation was purchased from Aalto Scientific, Ltd. San Marcos, CA.

## Solid-Phase RIAs

Hybridoma supernatant were assayed for specific antibody production in solid-phase RIA using cell extracts from different colon carcinomas, normal tissues, and purified CEA. Fifty $\mu$l containing 5 $\mu$g of cell extract or 20 ng of purified CEA were added to each well of 96-well polyvinyl microtiter plates and allowed to dry overnight at 37°C in a nonhumidified incubator. The wells were quenched to minimize nonspecific protein binding by the addition of 100 $\mu$l of 5% BSA in PBS containing calcium and magnesium (PBS with $Ca^{2+}$, $Mg^{2+}$). The BSA was aspirated after an incubation of 1 h at 37°C, and 50 $\mu$l of tissue culture supernatant were added to each well. After 1 h of incubation the unbound immunoglobulin was removed, and the plates were washed with 1% BSA in PBS. Each well was then incubated for 1 h at 37°C with $^{125}$I-labeled G $\alpha$ MulgG at 75,000 cpm in 25 $\mu$l. The unbound G $\alpha$ MulgG was aspirated, and the wells were washed extensively with PBS-1% BSA. The wells were then subjected to autoradiography using Kodak Xar film and DuPont Lightning Plus intensifying screens. The films were developed after an overnight exposure at 70°C. The bound $^{125}$I-G $\alpha$ MulgG was also detected by cutting the individual wells and measuring the radioactivity in a gamma counter.

## Fluorescence-activated Cell Sorting

Flow cytometry target cells were harvested and washed with $Ca^{2+}$, $Mg^{2+}$ -free Dulbecco's PBS. Cells ($10^6$) were incubated for 30 min at 4°C with 100 $\mu$l of hybridoma tissue culture supernatant. After incubation, the cells were washed and complete medium and further incubated with 100 $\mu$l of fluoresceinated goat anti-mouse immunoglobulin (Cappel Laboratories) for 30 min at 4°C. The cells were then washed and suspended at $10^6$ cells/ml in PBS with 2% fetal bovine serum. The cell suspensions were analyzed in an Ortho Cytofluorograf System 50 with an argon-ion laser tuned to 488 nm and emitting 200 mW of power. Dead cells were gated out by axial extinction, and one-dimensional histograms were analyzed on an Ortho Model 2150 computer system. The percentage of positive cells was calculated by setting a threshold of cells without primary antibody and subtracting the resulting value from the total percentage of antibody-stained cells.

## Western Blotting

Forty μg of cell extracts or 1 μg of purified CEA, diluted in SDS-PAGE sample buffer [0.125 M Tris-HCL(pH6.8)-4% SDS-20% glycerol-10% 2-mercapto-ethanol], was loaded onto a 5 to 20% linear gradient SDS-PAGE. After electrophoresis, proteins were transferred to nitrocellulose paper (0.45-μm pore size) at 4°C for 4 h at 30 V in transfer buffer [25 mM Tris-HCl (ph 8.3)-192 m glycine-20% methanol]. The blots were then incubated in PBS containing 5% BSA for 1 h at room temperature and washed with PBS containing 0.05% Tween-20. Ten ml of hybridoma tissue culture supernatant were added, and incubation continued for 1 h at room temperature with gentle agitation. After washing with PBS containing 0.05% Tween-20, the blots were incubated for 1 h at room temperature with $^{125}$I-labeled goat anti-mouse IgG (Kirkegard & Perry, Gaithersburg, MD.) The filters were then extensively washed overnight and exposed to Kodak Xar-5 X-ray film with DuPont Lightning Plus intensifying screen at -70°C for 2 h. For all experiments NS-1 tissue culture supernatant was used as a negative control.

## RESULTS

BALB/c mice were immunized with extracts of human primary and/or metastatic colon carcinoma following four different protocols as described. Sequential immunizations with three different tumors were also used in one of these protocols in an attempt to induce the production of antibodies to determinants shared by both metastatic and primary colon lesions. Furthermore, cell extract and partially purified membrane from the same primary colon carcinoma were used in two different immunization protocols. Splenocytes of immunized mice were fused with non-immunoglobulin-secreting NS-1 myeloma cells to generate 5052 primary hybridoma cultures. Supernatant fluids from these cultures were harvested and tested in solid-phase RIA to detect the presence of immunoglobulins reactive with extracts of their respective colon carcinoma immunogens and not with extracts of an apparently normal human spleen. The spleen used in the initial screening was previously shown to be reactive with some MAbs that are cross-reactive with CEA and CEA-related determinants. Whereas immunoglobulins from many cultures demonstrated reactivity with both spleen and colon carcinoma extracts, 341 cultures produced immunoglobulins reactive only with the respective colon carcinoma extract used. After double cloning of hybridoma cells by limiting dilution, the resultant culture supernatants were assayed in solidphase RIA for binding to extracts of five colon carcinoma biopsy specimens and several apparently normal human tissues, including four spleens, two kidneys, and three livers. Forty-nine MAbs were shown to be reactive with the five colon carcinoma extracts and nonreactive with any of the normal tissues. These MAbs were also shown to be nonreactive in solid-phase RIA with extracts of eleven preparations of RBC representing all major blood groups (A, B, O), as well as with extracts of normal human PMNs. In addition, the forty-nine MAbs were tested using the ABC-immunoperoxidase technique for reactivity to sections of five colon carcinomas and for lack of reactivity to sections of normal human colons, sweat gland, bone marrow, and lymph node. As a result of these initial screenings, fifteen MAbs, designated COL-1 to -15, were chosen for further characterization. The reactivities in solid-phase RIA of MAbs COL-1 to -15 with colon carcinoma extracts are shown in Table 1.

None of the MAbs was reactive with extracts of biopsy material from three normal adult livers and four normal adult spleens. Moreover all fifteen COL MAbs reacted in solid-phase RIA with five preparations of CEA obtained from different colon cancer patients.

Table 1

Reactivity of monoclonal antibodies in solid phase radioimmunoassays

| | Cell extracts [a] | | | | | | | CEA [b] | | | | | |
| --- | --- | --- | --- | --- | --- | --- | --- | --- | --- | --- | --- | --- | --- |
| | Colon carcinoma | | | | | Normal | | | | | | | |
| MAb | 1 | 2 | 3 | 4 | 5 | Liver (n=3) | Spleen (n=4) | 1 | 2 | 3 | 4 | 5 | Isotype [c] |
| COL-1 | 10.234[d] | 13.123 | 9.409 | 7.316 | 13.938 | Neg[e] | Neg | 23.562 | 19.816 | 12.959 | 24.273 | 24.548 | IgG2a |
| COL-2 | 8.815 | 13.325 | 7.769 | 6.798 | 10.148 | Neg | Neg | 24.111 | 16.587 | 15.174 | 22.139 | 25.874 | IgG2b |
| COL-3 | 7.404 | 9.019 | 8.459 | 4.502 | 10.223 | Neg | Neg | 7.406 | 6.162 | 3.760 | 8.623 | 4.920 | IgG1 |
| COL-4 | 10.737 | 12.767 | 8.911 | 6.534 | 14.079 | Neg | Neg | 28.428 | 21.987 | 26.253 | 30.117 | 31.064 | IgG2a |
| COL-5 | 1.400 | 2.589 | 1.112 | 1.165 | 1.895 | Neg | Neg | 2.256 | 1.677 | 2.225 | 3.435 | 3.677 | IgG2b |
| COL-6 | 11.429 | 12.822 | 9.298 | 8.983 | 13.391 | Neg | Neg | 13.644 | 11.493 | 9.730 | 12.101 | 15.411 | IgG1 |
| COL-7 | 8.856 | 10.338 | 9.023 | 4.916 | 11.359 | Neg | Neg | 10.333 | 11.698 | 5.693 | 9.981 | 11.195 | IgG1 |
| COL-8 | 7.885 | 9.261 | 7.511 | 3.182 | 9.735 | Neg | Neg | 11.485 | 9.943 | 10.451 | 13.516 | 12.040 | IgG1 |
| COL-9 | 3.436 | 4.225 | 3.330 | 1.470 | 4.638 | Neg | Neg | 4.908 | 7.107 | 6.070 | 8.672 | 8.760 | IgG2b |
| COL-10 | 7.440 | 9.164 | 8.922 | 4.465 | 9.978 | Neg | Neg | 11.335 | 8.361 | 5.194 | 9.449 | 6.555 | IgG1 |
| COL-11 | 9.020 | 12.576 | 9.098 | 7.951 | 10.294 | Neg | Neg | 13.061 | 9.956 | 6.336 | 7.996 | 11.574 | IgG1 |
| COL-12 | 6.970 | 7.802 | 5.827 | 3.751 | 7.975 | Neg | Neg | 11.123 | 8.461 | 7.509 | 9.111 | 9.226 | IgG1 |
| COL-13 | 4.556 | 3.634 | 4.180 | 1.531 | 5.435 | Neg | Neg | 8.930 | 8.149 | 8.051 | 5.740 | 5.440 | IgG1 |
| COL-14 | 8.083 | 8.126 | 8.545 | 4.762 | 6.526 | Neg | Neg | 8.846 | 8.776 | 2.403 | 10.880 | 9.603 | IgG1 |
| COL-15 | 5.799 | 6.268 | 4.665 | 2.761 | 6.230 | Neg | Neg | 11.193 | 8.558 | 9.275 | 9.963 | 10.293 | IgG1 |

[a] extracts prepared as described
[b] CEAs purified from five separate colon carcinoma patients.
[c] Isotypes determined as described
[d] cpm bound
[e] NEG, negative, in all cases but one (binding of COL-4 to spleens, 593 cpm), and cpm bound was less than 500 cpm. Individual values are not given (three livers and four spleens) for space considerations.

The differential reactivities of the MAbs to the colon cancer extracts and CEA preparations will be discussed below. All fifteen MAbs were of the IgG isotype of the 1, 2a, and 2b subclasses as shown in Table I.

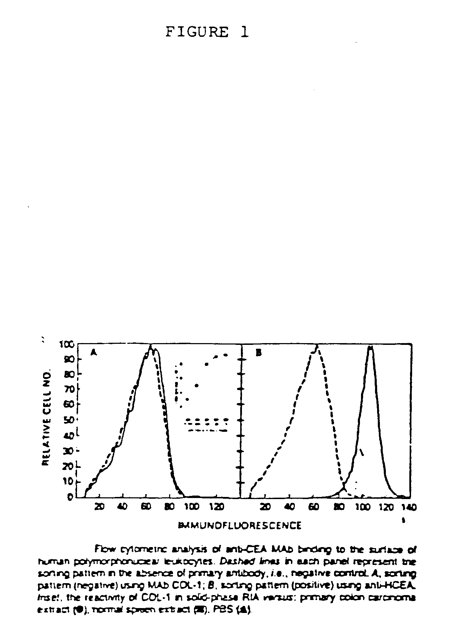

MAbs COL-1 to -15 were then tested by FACS for binding to the surface or normal human polymorphonuclear leukocytes; as discussed above, this has been the major cross-reactivity of anti-CEA monoclonals and heterologous sera. Bordes, M. et al. Eur. J. Cancer, 11:783-786 (1975). The reactivities of these COL MAbs were compared to that of a commercially available anti-HCEA. The results are shown in Figure 1. Dashed lines in each panel represent the sorting pattern in absence of primary antibody, i.e. negative control. (A, sorting pattern (negative) using COL-1; B, sorting pattern (positive) using anti-CEA).

As seen in Figure 1B, the anti-HCEA MAb reacted with the surface of over 90% of the human PMNs. All fifteen COL MAbs were nonreactive with the same preparation; a representative example (using MAb COL-1) of this nonreactivity to PMNs is shown in Figure 1A.

As an extension of these studies, MAb COL-1 was compared with MAb B1.1 for reactivity with various concentrations of an extract of PMNs. MAb B1.1 has previously been shown to be reactive with both purified CEA and CEA-related determinants. Herlyn, M. et al. Hybridoma, 23:329-339, 1983. Using 1.6 ng of purified MAbs COL-1 and B1.1, antigen dilution experiments revealed a positive reactivity using MAb B1.1 with as little as 100 ng of PMNs extract. On the other hand, addition of as much as 5000 ng of PMNs extract to reaction mixtures revealed no reactivity with MAb COL-1. Both B1.1 and COL-1 were equally positive for reactivity to purified preparations of CEA.

To further substantiate the results of Table 1 and Figure 1, an extract of a normal spleen known to contain PMNs reactive with an anti-CEA MAb was tested for reactivity with the COL MAbs. Figure 1, inset, shows the reactivity of COL-1 in a solid phase RI1 versus: primary colon carcinoma extract (●); normal spleen extract (■); PBS (▲). MAb COL-2 shows a high degree of selective reactivity for a colon carcinoma extract versus the extract of this spleen.

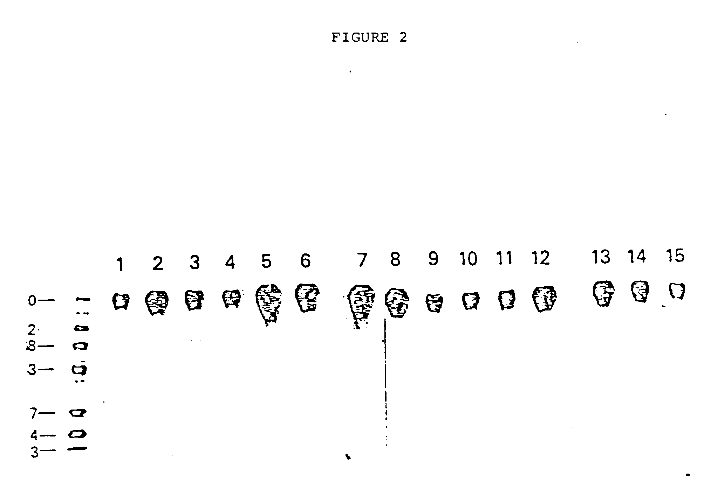

MAbs COL-1 to -15 were then characterized as to their ability to immunoreact with CEA in Western blotting experiments. In Figure 2, each lane presents the binding of a single COL MAb (COL-1 to COL-15) to a CEA preparation. Vertical scale is molecular weight markers (x10^{-3}). As seen in Figure 2, the fifteen COL MAbs immunodetected the $M_r$ 180,000 protein characteristic of the CEA molecule.

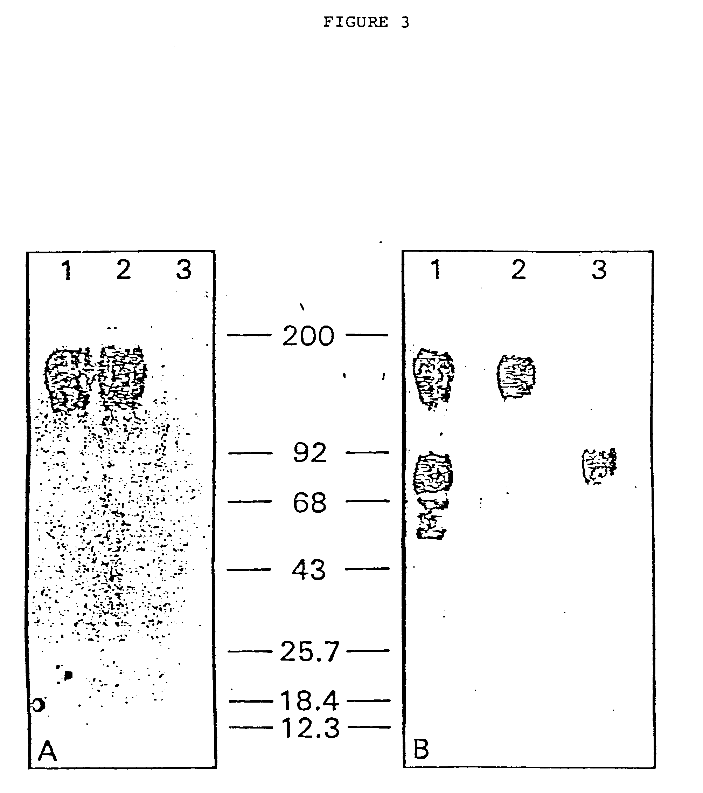

Previous studies have shown that other monoclonal antibodies immunoreactive with the purified $M_r$ 180,000 CEA molecule may also react with $M_r$ 160,000, 90,000, 50,000, and/or 40,000 proteins in colon cancer cells. Blaszczyk et al. Cancer Res., 44:245-253, 1984. These lower-molecular-weight molecules are believed to be the CEA cross-reacting antigens that are expressed on polymorphonuclear leukocytes and other normal tissues. An example of this type of cross-reactivity is shown in Figure 3B. (Proteins detected by MAb COL-1 (Figure 3A) and MAb B1.1 (Figure 3B) in extract of: Lane 1, metastatic colon carcinoma; Lane 2, primary colon carcinoma; Lane 3, normal human spleen; center scale, molecular weight markers (x10^{-3})). Extracts from one colon metastasis, one primary colon carcinoma, and a formal spleen were electrophoretically separated in SDS-PAGE gradient gels and transferred to nitrocellulose. After immunoblotting, MAb B1.1, previously shown to be reactive with CEA, detected the caracteristic $M_r$ 180,000, molecule present in the colon carcinoma extracts but also detected a $M_r$ 90,000 component present in the primary carcinoma as well as normal spleen extract. MAbs COL-1 to -15, on the other hand, detected only the $M_r$

180,000 CEA protein in extracts of colon carcinoma and did not detect any other component in the same spleen extract. This is exemplified by the result shown in Fig, 3A.

COL-1/3d6 Immunoenzymetric Assay

The 3d6 antibody was obtained from The Wistar Institute, Philadelphia, PA., and the COL-1 antibody was provided by Dr. Jeffrey Schlom of the National Cancer Institute, NIH, Bethesda Md.

The hybridoma cells were grown in tissue culture and were used to produce ascitic fluid in BALB/c mice primed with pristane (Aldrich Chemical Co., Milwaukee, WI 53201). Anti-CEA monoclonal immunoglobulin was purified from the ascites fluid by affinity chromatography, using protein A bound to Sepharose 4B (Pharmacia Fine Chemicals, Piscataway, N.J. 08854). The 3d6 antibody was eluted from the protein A column using 0.1M sodium citrate buffer at pH 4.0. COL-1 antibody was eluted from protein A using 0.1M sodium citrate buffer at pH 3.5. The purity of the isolated immunoglobulins was established by gel electrophoresis (sodium dodecyl sulfate/polyacrylamide gel electrophoresis), isoelectric focusing in agarose gel, and immunoelectrophoresis gels.

Purified 3d6 antibody was coated onto 1/4" polystyrene beads (Precision Plastic Ball Co., Chicago, IL 60641) and dried after treating with a PVP/Sucrose solution.

Purified COL-1 antibody was conjugated with HRP (Boehringer Mannheim Biochemicals, Indianapolis, IN) using a modified Nakane procedure. See Nakane P.K., Clinical Laboratory Techniques for the 1980's - (R.M. Nakamura and E.S. Tucker; Eds.) Alan R. Liss, Inc., New York, NY 10011 (1981).

HRP (10mg) was dissolved in 2.5 ml of $H_2O$ and oxidized with 0.05M sodium m-periodate for 20 minutes at room temperature. This solution was subsequently dialyzed against 0.001M sodium acetate buffer pH 4.4 at 4°C. After dialysis, 0.2M sodium carbonate buffer pH 9.5 was added to the HRP and immediately reacted with the COL-1 antibody (8 mg/ml in 0.01M sodium carbonate buffer pH 9.5). The reaction was incubated for 2 hours at room temperature, then reduced with 0.05M sodium borohydride (1.89 mg/ml in 0.01M sodium carbonate buffer pH 9.5). The reduced reaction mixture was dialyzed overnight at 4°C against 0.1M sodium phosphate buffer pH 7.5. Unbound HRP or COL-1 antibody was removed by gel permeation on a Sepharose 4BCL column (Pharmacia Fine Chemicals, Piscataway, NJ 08054). The purity of this conjugate was verified by high pressure liquid chromatography (HPLC).

CEA standards were prepared from supernatants of a cultured human colorectal adenocarcinoma derived from cell line SW 1116 (Leibovitz, A. et al., Cancer Res. 36, 4562-4569 (1976)) diluted into pooled human CEA-negative serum. These standards were calibrated against the World Health Organization Standard for CEA (National Institute for Biological Standards and Control, Holly Hill, London, NW3 6RB, U.K.)

All samples were assayed using a two site immunoenzymetric assay (IEMA) as follows:

0.2 ml of specimen or standard material was added to a reaction well containing one 3d6 coated polystyrene bead. The solid phase antibody and sample were allowed to incubate for 1 hour at 45°C. The bead was then washed and 0.2 ml of COL-1-HRP conjugate were added. The conjugate was allowed to react with the solid phase complex for 1 hour at 45°C. The bead was washed and transferred to a test tube. 0.5 ml of O-Phenylenediamine-2HCl substrate was added to the bead and incubated for 30 minutes at room temperature. The reaction was stopped by the addition of 1ml of 1N $H_2SO_4$. The reacted substrate solution tube was read in a spectrophotometer at $OD_{492}$. An assay standard curve was constructed by plotting the concentration of CEA standards versus $OD_{492}$. Experimental results were interpolated from the standard curve generated.

Table 2 shows the mean CEA values obtained for normal individuals and smokers. As expected, smokers exhibited higher CEA values than normal individuals. CEA levels in plasma and serum were substantially the same indicating that either blood components can be employed to assess CEA levels.

### Table 2

|  | Number | Mean CEA (ng/ml) |
|---|---|---|
| Smokers | 323 | 3.99±3.21 |
| Normals |  |  |
| plasma | 110 | 1.55±1.04 |
| serum | 82 | 1.24±0.77 |

Table 3 shows distribution of CEA levels in an NCI panel of serum from individuals with various malignant and benign diseases and from normal individuals.

### Table 3

|  | n | 0-3ng/ml | 3-5ng/ml | 5-10ng/ml | 10ng/ml |
|---|---|---|---|---|---|
| Colorectal Cancer | 70 | 8(11.4%) | 0 | 9(12.8%) | 53(75.7%) |
| Gastric Cancer | 10 | 7(70%) | 1(10%) | 1(10%) | 1(10%) |
| Pulmonary Cancer | 29 | 14(48.2%) | 7(24.1%) | 4(13.7%) | 4(13.7%) |
| Ovarian Cancer | 30 | 23(76.7%) | 3(10%) | 2(6.7%) | 2(6.7%) |
| Benign Colorectal | 64 | 45(70.3%) | 17(26.5%) | 1(1.42%) | 1(1.41%) |
| Benign Liver | 14 | 12(85.7%) | 2(14.3%) | 0 | 0 |
| Benign Ovarian | 28 | 27(96.4%) | 0 | 1(3.6%) | 0 |
| Benign Lung | 31 | 29(93.5%) | 1(3.2%) | 0 | 1(3.2%) |
| Healthy Controls | 85 | 62(72.9%) | 19(22.3%) | 2(2.3%) | 2(2.3%) |

More than 85% of patients with colorectal cancer show CEA levels greater than 5 ng/ml by the 3d6/COL-1 immunoenzymetric assay. Patients with other malignancies show a much smaller frequency of CEA levels in excess of 5 ng/ml. Patients with benign disease show a very low occurrence of CEA values above this level. Approximately 5% of normals showed CEA levels greater than 5 ng/ml. This may be attributable to smokers in the group, as normals in the panel were not screened for smoking history.

The data in Table 2 indicates that the 3d6/COL-1 immunoenzymetric assay has diagnostic value for colorectal cancer. If a level of CEA of 5 ng/ml or greater is chosen as an indication of colorectal cancer, based upon the data in table 3, more than 85% of patients with the disease can be identified by the test. Normal, non-smokers can be expected to show a false positive rate (CEA value greater than 5 ng/ml) of about 1/1000 based upon the statistical distribution of CEA values shown in Table 2. In order to reduce false positive results and enhance the predictive value of the assay, smokers among a normal population can be screened by a diagnostician.

The 3d6/COL-1 immunoenzymetric assay was compared with the Abbott CEA-EIA Monoclonal test. (Abbott Laboratories, North Chicago, Illinois) The Abbott assay was run following the manufacturer's standard protocol. In brief, the procedure entailed the following: 0.1 ml of sample or standard and 0.1 ml sample diluent were added to anti-CEA antibody coated beads. The mixture was incubated for one hour at 45°C. The beads were then washed three times with water. 0.2 ml anti-CEA-HRP solution was added to the bead and this combination was incubated for 1 hour at 45°C. The bead was washed three times with water. The beads were transferred to clear plastic tubes and 0.3 ml substrate was added. The enzyme reaction was allowed to proceed for 30 minutes at room temperature. The reaction was stopped by addition of 1N sulfuric acid. The enzyme reaction solution was read in a spectrophotometer at $OD_{492}$. The 3d6/COL-1 assay was run as described above with the exception of the dilution in the first incubation step.

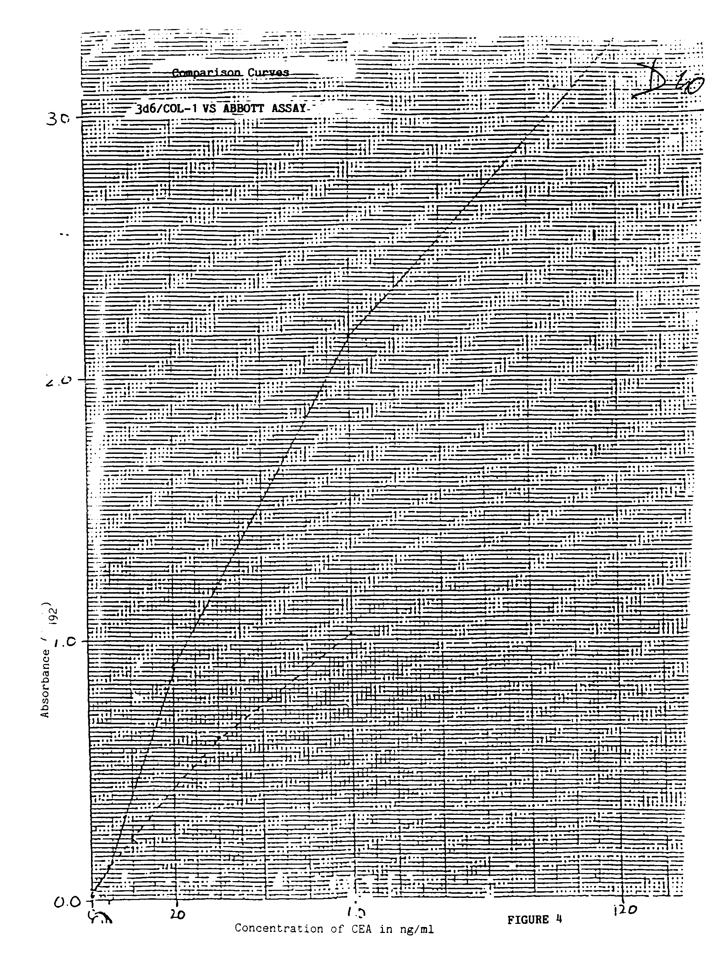

A plot of absorbance $A_{492}$ for CEA standards versus the corresponding CEA concentration (ng/ml) was constructed for each assay (Figure 4; ●----● Abbott assay; ●—● 3d6/COL-1 assay). As can be seen, the 3d6/COL-1 assay was more sensitive than the Abbott assay.

A comparison Abbott assay and the COL-1/3d6 IEMA was conducted with an NCI panel of serum from individuals with various malignant and benign diseases and from normal individuals. Table 4 shows the distribution of CEA levels as determined by each assay. CEA values as determined by each assay for the panel sera are compariable.

<center>TABLE 4</center>

| | | 0-3ng/ml | 3-5ng/ml | 5-10ng/ml | 10ng/ml |
|---|---|---|---|---|---|
| Ovarian Cancer | | | | | |
| n=29 | Abbott | 24/29 | 1/29 | 2/29 | 2/29 |
| | COL-1/3d6 | 23/29 | 3/29 | 1/29 | 2/29 |
| Lung Cancer | | | | | |
| n=19 | Abbott | 10/19 | 5/19 | 1/19 | 3/19 |
| | COL-1/3d6 | 10/19 | 3/19 | 3/19 | 3/19 |
| Colon Cancer | | | | | |
| n=12 | Abbott | 1/12 | 0 | 2/12 | 9/12 |
| | COL-1/3d6 | 1/12 | 0 | 3/12 | 8/12 |
| Gastric Cancer | | | | | |
| n=5 | Abbott | 3/5 | 0 | 1/5 | 1/5 |
| | COL-1/3d6 | 3/5 | 0 | 1/5 | 1/5 |
| Benign Lung | | | | | |
| n=11 | Abbott | 9/11 | 2/11 | 0 | 0 |
| | COL-1/3d6 | 9/11 | 1/11 | 0 | 1/11 |
| Benign Bowel | | | | | |
| n=6 | Abbott | 5/6 | 1/6 | 0 | 0 |
| | COL-1/3d6 | 6/6 | 0 | 0 | 0 |
| Benign Liver | | | | | |
| n=4 | Abbott | 4/4 | 0 | 0 | 0 |
| | COL-1/3d6 | 4/4 | 0 | 0 | 0 |
| Benign Ovarian | | | | | |
| n=2 | Abbott | 1/2 | 1/2 | 0 | 0 |
| | COL-1/3d6 | 2/2 | 0 | 0 | 0 |
| Healthy Controls | | | | | |
| n=12 | Abbott | 10/12 | 2/12 | 0 | 0 |
| | COL-1/3d6 | 10/12 | 2/12 | 0 | 0 |

Industrial Applicability

The immunometric assays for high molecular weight CEA of this invention can be used in the management and prognosis of colorectal cancers and for diagnosis of colorectal malignancies in "healthy" appearing individuals.

**Claims**

1. A solid phase sandwich immunometric assay for high molecular weight carcinoembryonic antigen employing (1) a solid phase to which is attached the 1116NS-3d (3d6) monoclonal antibody specific for the 180kD carcinoembryonic antigen, and (2) a soluble labelled antibody; characterised in that the soluble labelled antibody is the COL-1 monoclonal antibody specific for the 180kD carcinoembryonic antigen only, which antibody is obtainable from the hybridoma cell line having the deposit accession number ATCC HB 9238.

2. An immunometric assay for high molecular weight carcinoembryonic antigen (CEA) in a liquid sample, according to claim 1, comprising the steps of:
   a. forming an incubation mixture of the liquid sample and a solid phase immunoadsorbent containing the 3d6 anti-CEA monoclonal antibody;
   b. incubating the incubation mixture under conditions and for a period of time sufficient for the CEA in the liquid sample to bind to the immunoadsorbent;
   c. thereafter separating the immunoadsorbent from the liquid sample;
   d. forming an incubation mixture of the immunoadsorbent and the soluble, labeled COL-1 anti- CEA monoclonal antibody;

<center>12</center>

e. incubating the mixture under conditions and for a period of time sufficient for the labeled antibody to bind to the immunoadsorbent;

f. separating the solid phase immunoadsorbent from unbound labeled 3d6 anti-CEA antibody; and

g. detecting the amount of labeled monoclonal antibody bound to the immunoadsorbent or the amount of unbound labeled antibody; and

h. relating the amount of bound labeled monoclonal antibody or unbound labeled antibody detected to a predetermined quantitative relationship between the amount of labeled antibody and the amount of CEA in the liquid sample.

3. An immunoassay of Claim 1, wherein the solid phase immunoadsorbent comprises polystyrene beads with 3d6 anti-CEA monoclonal antibody conjugated thereto and the labeled, soluble antibody in the COL-1 anti-CEA monoclonal antibody.

4. An immunoassay of Claim 1, wherein the soluble COL-1 anti-CEA antibody is labeled with an enzyme, a radioisotope or a fluorescent compound.

5. An immunometric assay according to claim 1, which is a forward sandwich immunoenzymetric assay for carcinoembryonic antigen (CEA) in a blood plasma or serum sample, comprising the steps of:

a. forming an incubation mixture of the sample and a solid phase immunoadsorbent comprising polystyrene beads with the monoclonal 3d6 anti-CEA antibody affixed thereto;

b. incubating the mixture for about 1-4 hours at about room temperature to about 45°C;

c. thereafter separating the immunoadsorbent from the liquid sample;

d. forming an incubation mixture of the immunoadsorbent and soluble enzyme labeled COL-1 anti-CEA monoclonal antibody;

e. incubating the mixture for about 1-4 hours at room temperature to about 45°C;

f. separating the immunoadsorbent from unbound enzyme labeled COL-1 anti-CEA monoclonal antibody;

g. detecting the amount of enzyme labeled antibody bound to the immunoadsorbent; and

h. relating the amount of bound enzyme labeled COL-1 anti-CEA monoclonal antibody to a predetermined quantitative relationship between the amount of enzyme labeled anti-CEA antibody and the amount of CEA to determine the amount of CEA in the sample.

6. An immunometric assay for high molecular weight carcinoembryonic antigen (CEA) in a liquid sample according to claim 1, comprising the steps of:

a. forming an incubation mixture of the liquid sample and the soluble labeled COL-1 anti-CEA antibody;

b. incubating the incubation mixture under conditions and for a period of time sufficient for the CEA in the liquid sample to bind the labeled, soluble COL-1 monoclonal antibody;

c. contacting a solid phase immunoadsorbent containing the 3d6 anti-CEA monoclonal antibody with the incubation mixture under conditions and for a period of time sufficient for the CEA bound to the labeled, soluble COL-1 monoclonal antibody to bind the immunoadsorbent;

d. separating the solid phase immunoadsorbent from the incubation mixture;

e. detecting the amount of labeled COL-1 monoclonal antibody bound to the solid phase immunoadsorbent or the amount of unbound COL-1 labeled antibody; and

f. relating the amount of labeled monoclonal antibody detected to a predetermined quantitative relationship between the amount of labeled antibody and amount of CEA to determine the amount of CEA in the liquid sample.

7. An immunometric assay for high molecular weight carcinoembryonic antigen (CEA) in a liquid sample according to claim 1, comprising the steps of:

a. forming an incubation mixture containing:

i. liquid sample;

ii. a solid phase immunoadsorbent containing immobilized 3d6 monoclonal antibody; and

iii. labeled soluble COL-1 monoclonal antibody;

b. incubating said mixture under conditions and for a period of time sufficient for CEA in the liquid sample to complex with the immobilized 3d6 monoclonal antibody and labeled, soluble COL-1 monoclonal antibody;

c. thereafter separating said solid phase immunoadsorbent from the incubation mixture;

EP 0 225 709 B1

d. detecting the amount of labeled COL-1 monoclonal antibody bound to said solid phase immunoadsorbent or the amount of unbound labeled COL-1 antibody; and

e. relating the amount of labeled COL-1 monoclonal antibody detected to a predetermined quantitative relationship between the amount of labeled antibody and the amount of CEA to determined the amount of CEA in the liquid samples.

8. A kit containing reagents for performing a sandwich type immunometric assay for high molecular weight carcinoembryonic antigen (CEA) as defined in claim 1, comprising:

a. a solid phase immunoadsorbent containing 3d6 anti-CEA monoclonal antibody; and

b. a solution of labeled COL-1 anti-CEA monoclonal antibody.

9. A kit of reagents for performing an immunometric assay as defined in claim 1, said assay being a sandwich type immunoenzymetric assay for high molecular weight carcinoembryonic antigen (CEA), the kit comprising:

a. a polystyrene bead coated with 3d6 anti-CEA monoclonal antibody; and

b. a solution of horse radish peroxidase labeled COL-1 anti-CEA monoclonal antibody.

**Revendications**

1. Dosage immunométrique en "sandwich" (ou à double anticorps) et en phase solide, d'antigène carcinoembryonnaire de haute masse moléculaire, employant :

(1) une phase solide à laquelle est liée l'anticorps monoclonal 1116NS-3d(3d6) spécifique de l'antigène carcinoembryonnaire de 180kD, et

(2) un anticorps marqué soluble;

caractérisé en ce que l'anticorps marqué soluble est l'anticorps monoclonal COL-1 spécifique du seul antigène carcinoembryonnaire de 180kD, ledit anticorps pouvant être obtenu à partir de la lignée cellulaire hybridome dont le numéro d'accès au dépôt ATCC est HB 9238.

2. Dosage immunométrique d'antigène carcinoembryonnaire (CEA) de masse moléculaire élevée contenu dans un échantillon liquide selon la revendication 1, comprenant les étapes consistant à :

a) constituer un mélange pour incubation de l'échantillon liquide et d'un immunoadsorbant en phase solide contenant l'anticorps monoclonal 3d6 anti-CEA;

b) incuber le mélange pour incubation dans des conditions et pendant une période de temps suffisantes pour que le CEA contenu dans l'échantillon liquide se lie à l'immunoadsorbant;

c) séparer ensuite l'immunoadsorbant de l'échantillon liquide ;

d) constituer un mélange pour incubation de l'immunoadsorbant et de l'anticorps monoclonal COL-1 marqué soluble anti-CEA;

e) incuber le mélange dans des conditions et pendant une période de temps suffisantes pour que l'anticorps marqué se lie à l'immunoadsorbant;

f) séparer l'immunoadsorbant en phase solide de l'anticorps marqué 3d6 anti-CEA non lié; et

g) détecter la quantité d'anticorps monoclonal lié à l'immunoadsorbant ou la quantité d'anticorps marqué non lié; et

h) porter la quantité détectée d'anticorps monoclonal marqué lié ou de l'anticorps marqué non lié dans une relation quantitative prédéterminée entre la quantité d'anticorps marqué et la quantité de CEA, pour déterminer la quantité de CEA dans l'échantillon liquide.

3. Dosage immunométrique selon la revendication 1 dans lequel l'immunoadsorbant en phase solide comprend des billes ou perles de polystyrène auxquelles est conjugué l'anticorps monoclonal 3d6 anti-CEA, et l'anticorps soluble marqué dans l'anticorps monoclonal COL-1 anti-CEA.

4. Dosage immunométrique selon la revendication 1 dans lequel l'anticorps COL-1 soluble ANTI-CEA est marqué avec un enzyme, un radio-isotope ou un composé fluorescent.

5. Dosage immunométrique selon la revendication 1 du type dosage immunoenzymétrique en "sandwich avant" pour l'antigène carcinoembryonnaire (CEA) dans un échantillon de sérum de plasma sanguin, comprenant les étapes consistant à :

a) constituer un mélange pour incubation de l'échantillon et d'un immunoadsorbant en phase solide comprenant des billes ou perles de polystyrène auxquelles est fixé l'anticorps monoclonal 3d6 anti-

14

CEA;

b) incuber le mélange pendant une durée de 1 à 4 heures à une température allant environ de la température ambiante à environ 45°C;

c) séparer ensuite l'immunoadsorbant de l'échantillon liquide;

d) constituer un mélange pour incubation de l'immunoadsorbant et de l'anticorps monoclonal soluble COL-1 marqué par enzyme et anti-CEA;

e) incuber le mélange pendant une durée de 1 à 4 heures à une température allant environ de la température ambiante à environ 45°C;

f) séparer l'immunoadsorbant de l'anticorps monoclonal COL-1 non lié, marqué par enzyme et anti-CEA;

g) détecter la quantité d'anticorps marqué par enzyme lié à l'immunoadsorbant; et

h) porter la quantité d'anticorps monoclonal COL-1 lié, marqué par enzyme et anti-CEA, dans une relation quantitative prédéterminée entre la quantité d'anticorps marqué par enzyme, anti-CEA et la quantité de CEA pour déterminer la quantité de CEA dans l'échantillon.

6. Dosage immunométrique d'antigène carcinoembryonnaire (CEA) de masse moléculaire élevée contenu dans un échantillon liquide selon la revendication 1, comprenant les étapes consistant à:

a) constituer un mélange pour incubation de l'échantillon liquide et de l'anticorps soluble COL-1 marqué anti-CEA;

b) incuber le mélange pour incubation dans des conditions et pendant une période de temps suffisantes pour que le CEA contenu dans l'échantillon liquide se lie à l'anticorps monoclonal COL-1 soluble marqué;

c) mettre en contact un immunoadsorbant en phase solide contenant l'anticorps monoclonal 3d6 anti-CEA, avec le mélange pour incubation dans des conditions et pendant une période de temps suffisante pour que le CEA lié à l'anticorps monoclonal COL-1 soluble marqué se lie à l'immunoadsorbant;

d) séparer l'immunoadsorbant en phase solide du mélange pour incubation ;

e) détecter la quantité d'anticorps monoclonal COL-1 marqué lié à l'immunoadsorbant en phase solide ou la quantité d'anticorps COL-1 marqué non lié; et

f) porter la quantité d'anticorps monoclonal marqué détectée dans une relation quantitative prédéterminée entre la quantité d'anticorps marqué et la quantité de CEA pour déterminer la quantité de CEA dans l'échantillon liquide.

7. Dosage immunométrique d'antigène carcinoembryonnaire (CEA) de masse moléculaire élevée contenu dans un échantillon liquide selon la revendication 1, comprenant les étapes consistant à:

a) constituer un mélange pour incubation contenant

i) l'échantillon liquide

ii) un immunoadsorbant en phase solide contenant de l'anticorps monoclonal 3d6 immunobilisé; et

iii) de l'anticorps monoclonal COL-1 soluble marqué ;

b) incuber ledit mélange dans des conditions et pendant une période de temps suffisantes pour que le CEA contenu dans l'échantillon liquide forme un complexe avec l'anticorps monoclonal 3d6 immunobilisé et marqué, et l'anticorps monoclonal COL-1 soluble et marqué;

c) séparer l'immunoadsorbant en phase solide du mélange pour incubation ;

d) détecter la quantité d'anticorps monoclonal COL-1 marqué lié à l'immunoadsorbant en phase solide ou la quantité d'anticorps COL-1 marqué non lié; et

e) porter la quantité d'anticorps monoclonal marqué détectée dans une relation quantitative prédéterminée entre la quantité d'anticorps marqué et la quantité de CEA pour déterminer la quantité de CEA dans l'échantillon liquide.

8. Nécessaire contenant des réactifs pour effectuer un dosage immunométrique du type "sandwich" d'antigène carcinoembryonnaire (CEA) de masse moléculaire élevée, tel que défini à la revendication 1, comprenant

a) un immunoadsorbant en phase solide contenant de l'anticorps monoclonal 3d6 anti-CEA; et

b) une solution d'anticorps monoclonal COL-1 marqué anti-CEA

9. Nécessaire contenant des réactifs pour effectuer un dosage immunométrique tel que défini à la revendication 1, ledit dosage étant un dosage immunoenzymétrique du type "sandwich", d'antigène

EP 0 225 709 B1

carcinoembryonnaire (CEA) de masse moléculaire élevée, ledit nécessaire comprenant :
   a) une bille ou perles de polystyrène revêtue d'anticorps monoclonal 3d6 anti-CEA; et
   b) une solution d'anticorps monoclonal COL-1 anti-CEA et marqué par de la peroxydase du raifort.

**Patentansprüche**

1. Immunometrischer Festphasen-Sandwich-Test für hochmolekulargewichtiges karzinoembryonisches Antigen unter Verwendung (1) einer festen Phase, an der der monoklonale Antikörper 1116NS-3d (3d6), der für das karzinoembryonische Antigen 180kD spezifisch ist, angebracht ist, und (2) eines lösbaren markierten Antikörpers, dadurch gekennzeichnet, daß der lösbare markierte Antikörper der monoklonale Antikörper COL-1 ist, der nur für das karzinoembryonische Antigen 180kD spezifisch ist, welcher Antikörper von der die Hinterlegungsakzessionsnummer ATCC HB 9238 aufweisenden Hybridomzellinie erhältlich ist.

2. Immunometrischer Test für hochmolekulargewichtiges karzinoembryonisches Antigen (CEA) in einer flüssigen Probe nach Anspruch 1, mit den Schritten, daß
   a. ein Inkubationsgemisch der flüssigen Probe und eines Festphasenimmunoadsorbens, das den monoklonalen Antikörper 3d6 anti-CEA enthält, gebildet wird,
   b. das Inkubationsgemisch unter Bedingungen und für einen Zeitabschnitt inkubiert wird, die ausreichen, daß sich das CEA in der flüssigen Probe an das Immunoadsorbens bindet,
   c. danach das Immunoadsorbens von der flüssigen Probe getrennt wird,
   d. ein Inkubationsgemisch aus dem Immunoadsorbens und dem lösbaren markierten monoklonalen Antikörper COL-1 anti-CEA gebildet wird,
   e. das Gemisch unter Bedingungen und für eine Zeitdauer inkubiert wird, die ausreichen, daß sich der markierte Antikörper an das Immunoadsorbens bindet,
   f. das Festphasenimmunoadsorbens von ungebundenden markierten 3d6-anti-CEA-Antikörpern getrennt wird und
   g. die Menge markierter monoklonaler Antikörper, die an das Immunoadsorbens gebunden sind, oder die Menge ungebundender markierter Antikörper festgestellt wird, und
   h. die Menge festgestellter gebundener markierter monoklonaler Antikörper oder ungebundener markierter Antikörper in Beziehung gesetzt wird zu einem vorbestimmten quantitativen Verhältnis zwischen der Menge markierter Antikörper und der Menge von CEA, um die Menge von CEA in der flüssigen Probe zu bestimmen.

3. Immuntest nach Anspruch 1, bei dem das Festphasenimmunoadsorbens Polystyrolperlen mit daran konjugiertem monoklonalem Antikörper 3d6 anti-CEA und dem markierten, löslichen Antikörper im monoklonalen Antikörper COL-1 anti-CEA umfaßt.

4. Immuntest nach Anspruch 1, bei dem der lösbare COL-1-anti-CEA-Antikörper mit einem Enzym, einem Radioisotop oder einer fluoreszierenden Verbindung markiert wird.

5. Immunometrischer Test gemäß Anspruch 1, der ein immunoenzymetrischer fortschreitender Sandwich-Test für das karzinoembryonische Antigen (CEA) in einer Blutplasma- oder Serumprobe ist, mit den Schritten, daß
   a. ein Inkubationsgemisch von der Probe und einem Festphasenimmunoadsorbens mit Polystyrolperlen mit dem daran fixierten monoklonalen 3d6-anti-CEA-Antikörper gebildet wird,
   b. das Gemisch für etwa 1-4 Stunden bei etwa Raumtemperatur bis etwa 45°C inkubiert wird,
   c. danach das Immunoadsorbens von der flüssigen Probe getrennt wird,
   d. ein Inkubationsgemisch des Immunoadsorbens und des mit einem lösbaren Enzym markierten monoklonalen Antikörpers COL-1-anti-CEA gebildet wird,
   e. das Gemisch für etwa 1-4 Stunden bei etwa Raumtemperatur bis etwa 45°C inkubiert wird,
   f. das Immunoadsorbens von dem ungebundenen enzymmarkierten monoklonalen Antikörper COL-1-anti-CEA getrennt wird,
   g. die Menge des enzymmarkierten Antikörpers, die an das Immunoadsorbens gebunden ist, festgestellt wird und
   h. die Menge gebundener enzymmarkierter monoklonaler Antikörper COL-1-anti-CEA in Beziehung gesetzt wird zu einem vorbestimmten quantitativen Verhältnis zwischen der Menge enzymmarkierter Anti-CEA-Antikörper und der CEA-Menge, um die Menge des CEA in der Probe zu bestimmen.

16

**6.** Immunometrischer Test für ein hochmolekulargewichtiges karzinoembryonisches Antigen (CEA) in einer flüssigen Probe nach Anspruch 1, mit den Schritten, daß

a. ein Inkubationsgemisch der flüssigen Probe und des lösbaren markierten COL-1-anti-CEA-Antikörpers gebildet wird,

b. das Inkubationsgemisch unter Bedingungen und für einen Zeitabschnitt inkubiert wird, die ausreichen, daß sich das CEA in der flüssigen Probe an den gelösten markierten monoklonalen Antikörper COL-1 bindet,

c. ein den monoklonalen Antikörper 3d6 anti-CEA enthaltendes Festphasenimmunoadsorbens mit dem Inkubationsgemisch unter Bedingungen und für eine Zeitdauer kontaktiert wird, die ausreichen, daß das an den markierten, gelösten monoklonalen Antikörper COL-1 gebundene CEA das Immunoadsorbens bindet,

d. das Festphasen-Immunoadsorbens von dem Inkubationsgemisch getrennt wird,

e. die Menge von markierten monoklonalen Antikörpern COL-1, die an das Festphasenimmunoadsorbens gebunden sind, oder der Anzahl von ungebundenen markierten Antikörpern COL-1 festgestellt wird und

f. die Menge von markierten monoklonalen Antikörpern, die nachgewiesen sind, in Beziehung gesetzt wird zu einem vorbestimmten quantitativen Verhältnis zwischen der Menge von markierten Antikörpern und der Menge von CEA, um die Menge des CEA in der flüssigen Probe zu bestimmen.

**7.** Immunometrischer Test für hochmolekulargewichtiges karzinoembryonisches Antigen (CEA) in einer flüssigen Probe gemäß Anspruch 1, mit den Schritten, daß

a. ein Inkubationsgemisch gebildet wird, das

i. eine flüssige Probe,

ii. ein einen immobilisierten monoklonalen Antikörper 3d6 enthaltendes Festphasen-Immunoadsorbens und

iii. einen markierten lösbaren monoklonalen Antikörper COL-1 enthält,

b. das Gemisch unter Bedingungen und für einen Zeitabschnitt inkubiert wird, die ausreichen, daß das CEA in der flüssigen Probe mit dem immobilisierten monoklonalen Antikörper 3d6 und dem markierten, lösbaren monoklonalen Antikörper COL-1 einen Komplex bildet,

c. danach das Festphasenimmunoadsorbens vom Inkubationsgemisch getrennt wird,

d. die Menge von markierten monoklonalen Antikörpern COL-1, die an das Festphasenimmunoadsorbens gebunden sind, oder die Menge von ungebundenen markierten COL-1-Antikörpern festgestellt wird und

e. die Menge von markierten monoklonalen Antikörpern COL-1, die nachgewiesen sind, in Beziehung gesetzt wird zu einem vorbestimmten quantitativen Verhältnis zwischen der Menge von markierten Antikörpern und der Menge von CEA, um die Menge des CEA in den flüssigen Proben zu bestimmen.

**8.** Ausrüstung von Reagenzien zur Ausführung eines immunometrischen Tests vom Sandwichtyp für hochmolekulargewichtiges karzinoembryonisches Antigen (CEA), wie in Anspruch 1 angegeben, bestehend aus

a. einem Festphasenimmunoadsorbens, das monoklonale Antikörper 3d6 anti-CEA umfaßt, und

b. einer Lösung von markierten monoklonalen Antikörpern COL-1-anti-CEA.

**9.** Ausrüstung von Reagenzien für die Ausführung eines immunometrischen Tests gemäß Anspruch 1, der ein immunometrischer Test vom Sandwichtyp für hochmolekulargewichtiges karzinoembryonisches Antigen (CEA) ist, wobei die Ausrüstung

a. eine Polyestyrolperle, die mit monoklonalem Antikörper 3d6 anti-CEA überzogen ist, und

b. eine Lösung eines mit Meerrettichperoxidase markierten monoklonalen Antikörpers COL-1 anti-CEA umfaßt.

FIGURE 1

Flow cytometric analysis of anti-CEA MAb binding to the surface of human polymorphonuclear leukocytes. Dashed lines in each panel represent the sorting pattern in the absence of primary antibody, i.e., negative control. A, sorting pattern (negative) using MAb COL-1; B, sorting pattern (positive) using anti-HCEA. Inset, the reactivity of COL-1 in solid-phase RIA versus: primary colon carcinoma extract (●), normal spleen extract (▨), PBS (▲).

FIGURE 2

FIGURE 3

FIGURE 4